(19) Europäisches Patentamt
European Patent Office
Office européen des brevets

(11) Numéro de publication: **0 140 786**
**B1**

(12) # FASCICULE DE BREVET EUROPÉEN

(45) Date de publication du fascicule du brevet:
20.07.88

(51) Int. Cl.⁴: **A 61 B 17/60**

(21) Numéro de dépôt: **84402115.4**

(22) Date de dépôt: **22.10.84**

(54) Appareils orthopediques pour immobiliser un os fracturé.

(30) Priorité: **27.10.83 FR 8317361**

(43) Date de publication de la demande:
**08.05.85 Bulletin 85/19**

(45) Mention de la délivrance du brevet:
**20.07.88 Bulletin 88/29**

(84) Etats contractants désignés:
**BE CH DE FR GB IT LI NL**

(56) Documents cité:
**CH-A-638 390**
**FR-A-2 174 377**
**FR-A-2 312 226**
**FR-A-2 442 044**
**FR-A-2 457 676**
**US-A-1 997 466**

(73) Titulaire: **SOCIETE DE REALISATIONS ELECTROMECANIQUES (S.O.R.E.M.), Zone Industrielle Toulon- Est, F-83088 Toulon (FR)**

(72) Inventeur: **Ingrassia, Vincent, 201 Route Lentisques Parc des Oiseaux, F-83400 Hyeres (FR)**
Inventeur: **Meyrueis, Jean- Paul, 4 rue de Metz, F-83200 Toulon (FR)**
Inventeur: **( )**

(74) Mandataire: **Ores, Irène, CABINET ORES 6, Avenue de Messine, F-75008 Paris (FR)**

LIBER, STOCKHOLM 1988

## Description

La présente invention a pour objet des appareils orthopédiques servant à la fois à immobiliser un os fracturé et à réduire une fracture.

On connaît des appareils du type attelles ou éclisses servant à immobiliser un membre, supérieur ou inférieur, dont un os a été fracturé, pour permettre de le transporter.

On connaît également des fixateurs externes qui comportent un support rigide placé à l'extérieur d'un membre et relié à l'os par des broches. Les Brevets US-2 435 850 (J.R. SIEBRANDT) et FR-1 569 090 (P. et A. GERARD) décrivent de tels appareils.

On connaît également des fixateurs externes comportant des broches autoforantes qui traversent un os fracturé de part et d'autre de la fracture et qui sont montées sur un support rigide en deux parties qui sont réunies par des moyens qui permettent de déplacer légèrement les broches afin de réduire la fracture, puis de les immobiliser solidement pendant la consolidation.

La Demande de Brevet FR-A-2 457 676 décrit un fixateur externe qui comporte des broches autoforantes qui traversent l'os de part en part.

Ces broches sont fixées temporairement, pendant le transport, sur un premier tube rigide externe portant des rangées de trous. Après réduction de la fracture, les broches sont fixées sur deux bouts de tubes perforés, externes au membre, qui sont montés sur des rotules fixées par des colliers sur un tube rigide. Cet appareil fixateur externe connu peut être facilement adapté à des fractures déformantes.

Grâce aux tubes perforés portant les broches qui sont reliés au tube fixateur externe par des rotules et des colliers coulissants, il permet d'obtenir un déplacement relatif des deux parties de l'os situées de part et d'autre de la fracture et donc de réduire celle-ci.

Toutefois, cet appareil ne procure pas une bonne contention de l'os pendant la consolidation, à cause des rotules.

L'objectif de la présente invention est de procurer des fixateurs externes à broches plus perfectionnées, qui permettent de régler la compression ou l'extension de l'os pendant la consolidation, qui permettent d'obtenir une très bonne rigidité dans toutes les directions et qui comportent des broches autoforantes qui sont vissées seulement dans l'os fracturé sans traverser le membre de part en part.

La présente invention a pour objet un appareil orthopédique pour immobiliser un os fracturé du type comportant au moins un fixateur externe rigide, constitué par un tube perforé qui est placé à l'extérieur et au contact d'un membre ayant un os fracturé, et des broches autoforantes qui sont vissées dans l'os fracturé de part et d'autre de la fracture et qui sont engagées dans les perforations dudit tube et bloquées dans celui-ci par des vis de blocage, caractérisé en ce ledit fixateur externe comprend deux tubes perforés distincts disposés selon le même axe, dans le prolongement l'un de l'autre et reliés entre eux par un tube de liaison, en ce que l'un des deux tubes perforés est solidarisé avec le tube de liaison pour être fixe, tandis que l'autre tube perforé peut être déplacé axialement le long du tube de liaison, par rapport au tube perforé fixe, en ce que le tube de liaison porte un filetage exterieur au moins à ses deux extrémités opposées et en ce que deux écrous sont prévus respectivement aux extrémités filetées du tube de liaison, au voisinage des extrémités correspondantes du tube perforé mobile, de manière à permettre le déplacement du tube perforé mobile le long du tube de liaison et à bloquer ledit tube mobile dans la position voulue, par vissage desdits écrous sur lesdites extrémités filetées du tube de liaison.

Selon un mode de réalisation avantageux de l'appareil conforme à l'invention, le tube perforé fixe est solidarisé avec le tube de liaison par vissage de l'une de ses extrémités filetées dans le filetage interne prévu à l'extrémité correspondante du tube perforé fixe ou par soudage de leurs extrémités adjacentes, ou en réalisant le tube perforé fixe et le tube de liaison en une seule pièce, venue de fabrication.

Selon un autre mode de réalisation avantageux de l'appareil conforme à l'invention, le tube de liaison comporte deux fentes longitudinales diamétralement opposées à travers lesquelles passent lesdites broches et une ou deux fentes longitudinales situées dans un plan perpendiculaire au plan des deux premières fentes dans l'une desquelles les vis de blocage des broches sont engagées.

Avantageusement, un appareil orthopédique selon l'invention comporte un deuxième fixateur externe qui est placé à l'extérieur du membre fracturé, parallèlement au premier, coopère avec celui-ci et est orienté de telle sorte que les broches engagées dans les deux fixateurs soient situées dans deux plans faisant entre eux un angle compris entre 60° et 120° et les deux fixateurs sont reliés entre eux par des tiges transversales.

Conformément à l'invention, le deuxième fixateur externe est identique au premier fixateur ou est constitué par un tube perforé unique.

Selon un mode de réalisation avantageux de l'invention, chacun desdits premier et deuxième fixateurs porte deux colliers coulissants, et lesdites tiges transversales sont engagées et bloquées dans deux colliers situés respectivement sur chacun des deux fixateurs, sensiblement au même niveau.

Selon un autre mode de réalisation avantageux de l'invention, l'appareil orthopédique peut comporter un troisième fixateur rigide, qui est situé à l'extérieur d'une autre partie dudit membre comportant un deuxième os fracturé et qui est relié à l'un desdits premier ou deuxième fixateurs situés à l'extérieur d'une première partie dudit membre comportant un premier os fracturé, par l'intermédiaire d'une rotule située au niveau de l'articulation entre les deux os fracturés.

Selon encore un autre mode de réalisation de l'appareil orthopédique conforme à l'invention comprenant deux fixateurs associés par des colliers, chaque collier se compose de deux parties sensiblement symétriques articulées entre elles et d'un dispositif de blocage de chaque collier en position voulue sur le tube perforé correspondant.

Selon une disposition avantageuse de ce mode de réalisation, chacun des colliers se compose de deux demi-mâchoires articulées entre elles par une articulation à charnière et le mors de l'une des demi-mâchoires porte une vis, notamment du type imperdable, et le mors de l'autre demi-mâchoire porte un écrou qui coopère avec ladite vis pour bloquer ledit collier en position sur un tube.

Selon une autre disposition avantageuse de ce mode de réalisation, le mors de la demi-mâchoire qui porte l'écrou de blocage comporte sur sa face externe un dégagement pour loger la tête de l'écrou de blocage.

Selon encore une autre disposition avantageuse de ce mode de réalisation, la tête de l'écrou de blocage comporte un orifice traversant ménagé perpendiculairement à l'axe dudit écrou et destiné à recevoir une douille propre à loger, notamment, une broche destinée à immobiliser, dans le cas, en particulier, d'une fracture multiple, un fragment d'os se trouvant hors du système de réduction du fixateur.

Selon un mode de réalisation avantageux de l'appareil orthopédique conforme à l'invention, celui-ci comprend deux colliers du type défini dans ce qui précède, montés par leurs mors sur un axe commun, les faces adjacentes des mors voisins de chaque collier comportant chacune sur le pourtour des orifices de passage dudit axe, un crantage d'accouplement desdits colliers en position bloquée, et ledit axe étant pourvu d'un écrou ou analogue de serrage en position, des mors des colliers sur leur axe commun, pour bloquer les colliers dans n'importe quelle position relative l'un par rapport à l'autre.

Selon un autre mode de réalisation de l'appareil orthopédique conforme à la présente invention, la rotule qui relie le troisième fixateur au premier ou au deuxième fixateur, comprend deux parties orientables l'une par rapport à l'autre.

De préférence, les surfaces en regard de chacune des deux parties dont se compose ladite rotule, qui sont sensiblement constituées par deux demi-sphères, sont des surfaces crantées pour assurer le blocage mutuel desdites deux parties.

Conformément à une disposition préférée de l'invention, les deux parties qui composent la rotule sont mobiles par rotation l'une par rapport à l'autre autour d'un axe d'assemblage perpendiculaire à leurs surfaces de jonction.

Conformément à une autre disposition préférée de l'invention, chacune des deux parties dont se compose la rotule comporte un embout de fixation dont chacun est destiné à être inséré dans l'une des extrémités de tubes voisins à relier suivant une orientation angulaire relative voulue.

Egalement conformément à l'invention, chaque embout comporte une gorge périphérique qui coopère avec une vis de blocage insérée dans l'un des trous ménagés sur la paroi d'un tube à relier, au voisinage de son extrémité, pour bloquer ledit embout en position dans l'extrémité dudit tube et l'embout présente sur sa face tournée vers la partie de la rotule à laquelle il est associé, un dégagement propre à permettre la rotation de ladite partie de la rotule sans frottement.

Selon une autre disposition préférée de l'invention, l'embout est relié à la partie de la rotule à laquelle il est associé, par un segment de collerette comportant un méplat sensiblement dans le prolongement de la surface de jonction de ladite partie avec la surface en regard de l'autre partie de la rotule.

Selon un autre mode de réalisation de l'appareil orthopédique, la rotule se compose de trois parties, à savoir les deux parties semi-sphériques mobiles par rotation l'une par rapport à l'autre, définies dans ce qui précède, et une pièce intermédiaire annulaire de même diamètre que lesdites deux demi-sphères, dont les deux faces opposées sont également crantées et qui porte également un embout tel que défini plus haut.

Ainsi que cela ressort de ce qui précède, grâce au mouvement relatif axial de l'un des tubes du fixateur externe par rapport à l'autre, les appareils conformes à la présente invention permettent de mettre l'os fracturé en compression ou en élongation, en cours de consolidation, sans avoir à déplacer les broches.

En outre, les appareils conformes à la présente invention comportant deux fixateurs externes réunis par des tiges transversales, assurent une très bonne contention de l'os, en toutes circonstances et peuvent permettre les mouvements du membre fracturé pendant la période de consolidation de l'os.

Les appareils selon l'invention comportant deux fixateurs externes qui comprennent chacun deux tubes déplaçables axialement et qui sont réunis par des tiges transversales, permettent de régler la compression ou l'élongation de l'os pendant la consolidation sans avoir à extraire les broches de l'os tout en assurant un très bon maintien en place de l'os fracturé.

L'association de deux fixateurs entre eux par l'intermédiaire d'une rotule permet d'articuler entre eux deux fixateurs dans un champ angulaire très large et, dans le cas où la rotule est en trois parties, une telle réalisation permet d'articuler entre eux trois tubes par l'une de leurs extrémités, notamment dans les cas d'arthrodèse du pied.

Les colliers peuvent être mis en place très facilement en n'importe quel point du tube sur lequel ils sont montés, sans avoir à démonter quelque pièce que ce soit.

L'association de deux colliers sur un axe commun, telle que définie plus haut, constitue une solution plus souple que la rotule car elle permet une rotation des tubes accouplés par les colliers, de 360° l'un par rapport à l'autre, avec une mobilité des deux tubes. L'association de deux colliers telle que définie plus haut permet le décalage des tubes fixateurs, ce qui présente un grand intérêt dans les montages où l'on a besoin d'une rigidité totale et où l'encombrement de ces montages, dû au décalage des tubes, est tolérable.

L'association de deux colliers permet une adaptation aux besoins successifs qui apparaissent chez un même malade, sans avoir à changer d'appareillage, car l'un des tubes associés par les colliers peut, à volonté, être mis en position décalée haute ou basse, par exemple en fonction des problèmes du moment, tels qu'enflure, qui nécessite le montage temporaire en partie haute et désenflure, qui permet le montage en partie basse, en tenant compte de la donnée chirurgicale traumatique fondamentale, selon laquelle le fixateur doit être le plus proche possible de l'os à réduire.

Outre les dispositions qui précèdent, l'invention comprend encore d'autres dispositions, qui ressortiront de la description qui va suivre.

L'invention sera mieux comprise à l'aide du complément de description qui va suivre, qui se réfère aux dessins annexés dans lesquels:

- la figure 1 est une vue en élévation d'un fixateur conforme à l'invention monté sensiblement parallèlement à un fémur fracturé, avec le fixateur au contact de la cuisse,

- la figure 2 est une coupe axiale du fixateur selon la figure 1,

- la figure 3 est une vue éclatée partielle en perspective du fixateur selon la figure 1,

- la figure 4 est une vue en élévation d'un deuxième mode de réalisation d'un appareil orthopédique selon l'invention, dont

- la figure 5 est une coupe transversale selon V-V de la figure 4,

- les figures 6 et 7 sont des vues respectivement en plan et éclatée d'un mode de réalisation de la rotule de liaison entre deux fixateurs,

- les figures 8 et 9 sont des vues respectivement en plan et éclatée, d'un autre mode de réalisation de la rotule de liaison,

- la figure 10 est une vue en plan d'un mode de réalisation du collier de fixation des broches,

- la figure 11 est une vue en plan d'un ensemble de deux colliers d'assemblage de tubes de fixateurs,

- la figure 12 est une vue en plan d'une douille de réduction associée à un collier de fixation des tiges, dans laquelle est montée une broche, et

- la figure 13 est une vue en coupe suivant XIII-XIII de la figure 12.

Il doit être bien entendu, toutefois, que ces dessins et les parties descriptives correspondantes, sont donnés uniquement à titre d'illustration de l'objet de l'invention, dont ils ne constituent en aucune manière une limitation.

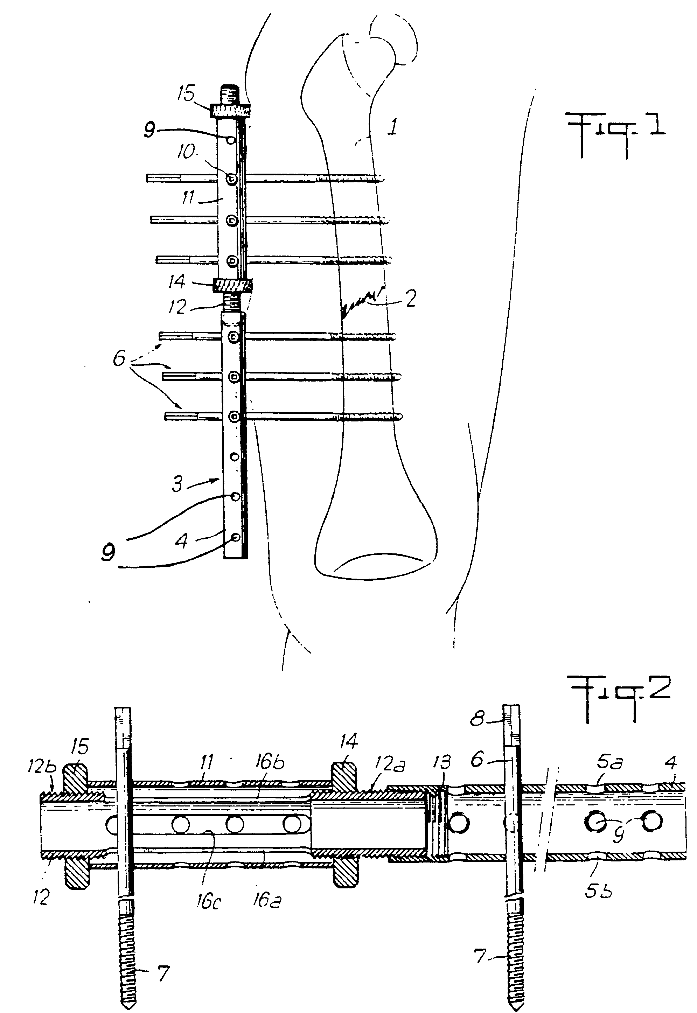

La figure 1 représente le contour d'une cuisse droite vue de face et le fémur 1, qui a été fracturé, en 2.

Le fémur est représenté après que la fracture a été réduite.

Un appareil selon l'invention comporte un fixateur externe rigide 3 qui est placé le long de la cuisse à l'extérieur de la face externe de celle-ci. Le fixateur externe 3 présente la forme d'un tube allongé qui est placé dans une position sensiblement parallèle à celle de l'os fracturé.

Le fixateur 3 comporte un premier tube 4 qui porte des rangées de trous diamétralement opposés 5a, 5b. Des broches autoforantes 6, en métal inoxydable, ayant un diamètre de l'ordre de 5 mm, peuvent être engagées dans une paire de trous alignés 5a, 5b.

Les broches 6 ont une extrémité munie d'un filetage 7 autoforant qui se visse dans l'os. L'autre extrémité des broches comporte une tête polygonale 8 qui permet de faire tourner les broches avec une clef pour les visser dans l'os fracturé.

Le tube 4 comporte également deux rangées de trous filetés diamétralement opposés 9, qui sont disposés en croix par rapport aux perforations 5a, 5b et dans les mêmes plans transversaux que celles-ci.

Les trous filetés 9 reçoivent des petites vis 10 de blocage des broches.

Avantageusement, les vis 10 et les broches 6 comportent des têtes creuses et sont manoeuvrées au moyen d'une même clef alêne à tête pleine polygonale.

On voit sur la figure 1 que les broches 6 sont vissées dans l'os et traversent celui-ci de part en part, sans traverser le membre fracturé de part en part.

Le fixateur externe 3 comporte un deuxième tube 11 analogue au premier.

Les deux tubes 4 et 11 peuvent être de même longueur ou non. Les fixateurs ayant deux tubes de même longueur sont utilisés pour les fractures situées dans la partie médiane du fémur. Pour les fractures situées dans les parties hautes ou basses du fémur, on utilise un fixateur dissymétrique comportant un tube 11 plus court que le tube 4.

Le tube 11 est monté coulissant sur un troisième tube 12 ayant deux extrémités comportant un filetage externe 12a et 12b.

Une extrémité du tube 4 comporte un filetage interne 13 dans lequel l'extrémité 12a est vissée.

L'appareil comporte deux écrous 14 et 15 qui sont vissés respectivement sur les extrémités filetées 12a et 12b, de part et d'autre du tube 11, et qui permettent de déplacer axialement le tube 11 et de le bloquer dans une position déterminée.

Le tube 12 comporte quatre fentes longitudinales 16a, 16b, 16c, 16d qui sont disposées en croix et qui s'étendent sur une grande partie de la longueur du tube 11. La largeur des fentes 16 est supérieure au diamètre des broches 6 et des vis de blocage 10, de sorte que celles-ci peuvent passer à travers les fentes 16. Les fentes 16 permettent que les broches et les vis engagées dans le tube 11 puissent coulis ser axialement en même temps que le tube 11.

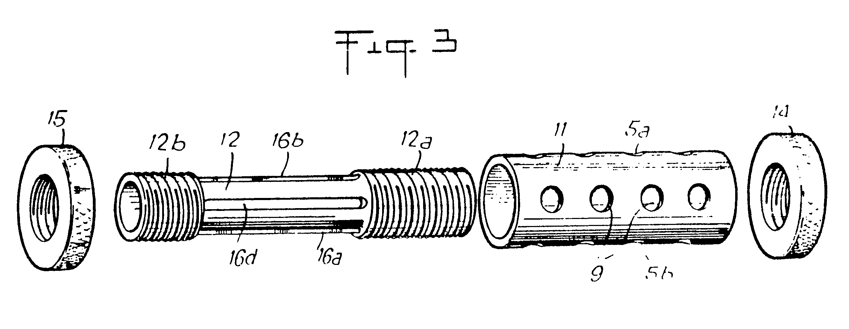

Un appareil selon les figures 1 et 2 est utilisé pour maintenir un os immobile pendant la consolidation d'une fracture ouverte ou que l'on ouvre volontairement pour réduire la fracture. Après avoir amené les fragments d'os à coïncider, on met en place le tube fixateur 3 et on engage les broches 6 à travers les orifices des deux tubes 4 et 11, puis on visse les extrémités autoforantes dans les deux parties de l'os situées de part et d'autre de la fracture 2, comme on le voit sur la figure 1. On bloque les broches dans cette position au moyen de vis de blocage 10. Lorsqu'on le désire, en cours de consolidation de l'os, mettre celui-ci en compression, on dévisse l'écrou 14 et on visse l'écrou 15, qui repousse le tube coulissant 11 vers le tube 4. Le déplacement du tube 11 entraîne une des parties de l'os à se déplacer vers l'autre. Inversement, si l'on désire mettre l'os en élongation, on dévisse l'écrou 15, puis on visse l'écrou 14 pour éloigner le tube 11 du tube fixe 4.

Les appareils selon les figures 1 et 2 permettent de placer les deux broches qui sont de part et d'autre de la fracture, à proximité de celle-ci.

La figure 3 est une vue éclatée en perspective du troisième tube 12, sur laquelle on voit trois des quatre fentes longitudinales 16a, 16b et 16d. On voit également sur cette figure le deuxième tube coulissant 11 comportant des perforations 5a, 5b pour le passage des broches, et des perforations 9 pour le passage des vis de blocage et on voit de plus les deux écrous moletés 14 et 15 qui se vissent sur les extrémités filetées 12a et 12b du troisième tube 12, de part et d'autre du tube 11.

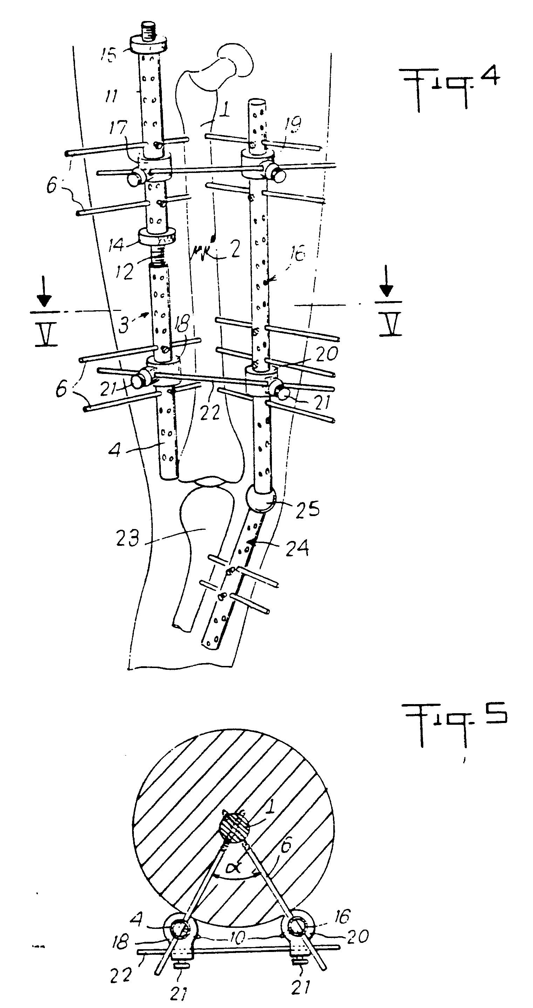

Les figures 4 et 5 représentent un autre mode de réalisation d'un appareil selon l'invention.

La figure 4 est une vue de côté d'une cuisse droite dont le fémur 1 présente une fracture 2.

L'appareil selon la figure 2 comporte un premier fixateur externe 3, qui est identique au fixateur décrit sur la figure 1 et dont les parties homologues sont représentées par les mêmes repères.

L'appareil selon la figure 4 comporte un deuxième fixateur externe qui est constitué par un tube rigide 16 comportant des perforations diamétralement opposées pour le passage des broches autoforantes 6, et d'autres perforations, situées en croix par rapport aux précédentes pour le passage des vis de blocage des broches 6.

Sur chacun des tubes 4 et 11 du premier fixateur est monté un collier coulissant 17 et 18.

Deux colliers coulissants identiques 19 et 20 sont montés sur le tube 16.

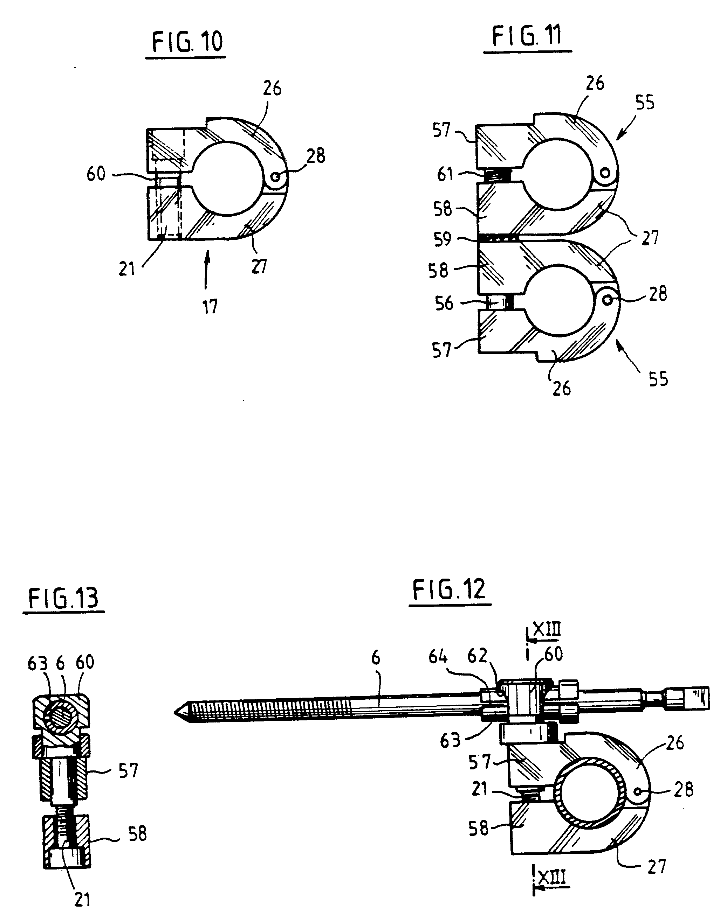

Chaque collier (cf. figure 10) est composé de deux parties ou demi-mâchoires 26, 27, articulées entre elles par une charnière 28, et comporte une vis de blocage 21 portée par la partie 27 du collier, telle qu'une vis imperdable, par exemple, qui coopère avec un écrou 60 porté par la partie 26 du collier, pour desserrer le collier et le faire coulisser ou pivoter sur le tube, puis le bloquer dans une position déterminée.

Les vis de blocage 21 comportent un alésage qui les traverse de part en part, dans lequel on engage une tige 22 qui relie ensemble deux colliers placés sensiblement à la même hauteur sur les deux fixateurs externes.

La figure 5 est une coupe transversale selon V-V de la figure 4. On voit sur cette coupe que les deux fixateurs externes sont orientés de telle manière que les broches 6 qui les traversent respectivement fassent entre elles un angle α qui est compris entre 60° et 120°. On voit également que les colliers 18 et 20 sont orientés de telle manière que les tiges 22 qui relient deux colliers forment le troisième côté d'un triangle dont les broches 6 forment les deux autres côtés.

Grâce à cette disposition, l'os est relié par les broches aux deux fixateurs qui sont reliés entre eux par les tiges 22. On obtient ainsi une structure en triangle très rigide.

Après avoir réduit la fracture, on met en place le premier fixateur 3 et les broches qui l'équipent et au moyen des écrous 14 et 15, on règle la compression ou l'élongation de l'os fracturé, après quoi on met en place le deuxième fixateur 16 et les broches qui l'équipent, puis on relie les deux fixateurs par les tiges 22 que l'on bloque dans les colliers. On obtient ainsi un appareil très rigide dans toutes les directions et indéformable, qui immobilise très solidement le membre fracturé pendant toute la période de consolidation.

Selon une variante de réalisation, un appareil selon la figure 4 peut comporter deux fixateurs externes identiques au fixateur 3 de la figure 1, c'est-à-dire deux fixateurs comportant chacun un tube fixe 4 et un tube coulissant 11, chacun de ces deux tubes portant un collier permettant de relier respectivement les tubes fixes et les tubes coulissants par une tige transversale 22.

Cette variante présente l'avantage que l'on peut éloigner ou rapprocher simultanément les deux tubes coulissants des deux tubes fixes et donc régler la compression ou l'élongation de l'os fracturé en cours de consolidation.

Un appareil selon la figure 4 peut comporter éventuellement un troisième fixateur externe 24 qui est placé le long du tibia et qui est relié à l'un des fixateurs placés le long de la cuisse par une rotule 25 située au niveau du genou, qui sera décrite plus en détail plus loin. Le fixateur 24 est un tube perforé dans lequel on engage et on bloque des broches autoforantes 6 qui sont vissées dans le tibia 23. Cette variante permet d'immobiliser simultanément deux os situés de part et d'autre d'une articulation, par exemple le fémur et le tibia, sans bloquer l'articulation.

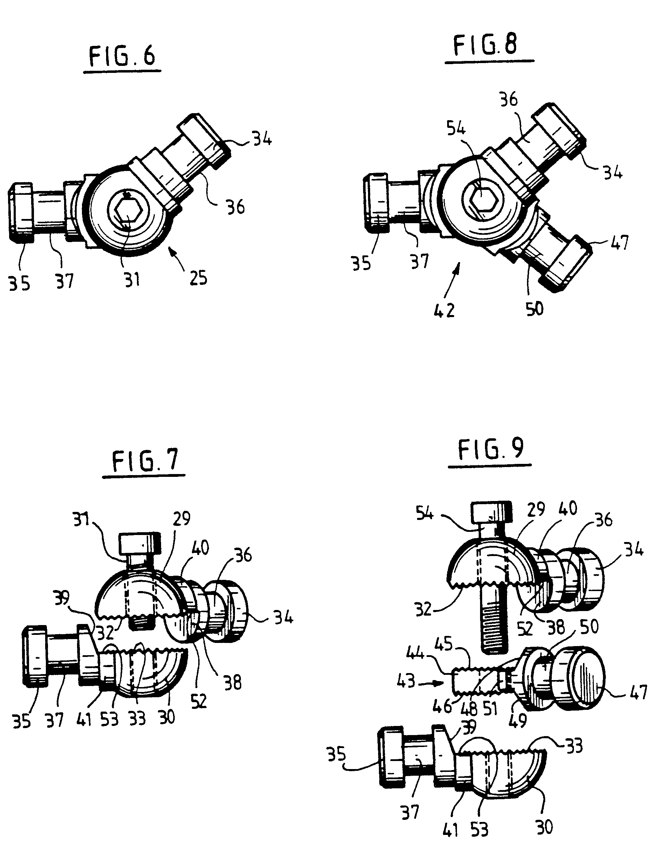

La rotule 25 (cf. figures 6 et 7) se compose de deux parties, essentiellement des demi-sphères 29, 30, qui sont assemblées au moyen d'un axe 31 autour duquel elles sont mobiles l'une par rapport à l'autre, par rotation.

Les deux faces planes 32, 33 en regard l'une de l'autre, de chaque demi-sphère 29, 30 sont crantées pour assurer le blocage des deux demi-sphères assemblées pour former la rotule 25.

Chacune des deux demi-sphères 29, 30 porte un embout 34, 35 destiné à être inséré dans deux tubes adjacents (4, 16, 24, etc...) de deux fixateurs que l'on souhaite assembler de façon articulée l'un à l'autre. Chaque embout 34, 35 comporte une gorge périphérique 36, 37 qui reçoit l'extrémité d'une vis de blocage (non représentée) insérée dans l'un des orifices ménagés sur la paroi du tube (4, 16, 24, etc...) à assembler, à proximité de l'extrémité de ce dernier adjacente à la rotule 25. La face de l'embout tournée vers la demi-sphère comporte un dégagement angulaire 38, 39, qui permet la rotation des demi-sphères 29, 30 l'une par rapport à l'autre sans frottement. En outre, chaque demi-sphère 29, 30 comporte un segment de collerette 40, 41 qui relie la demi-sphère concernée à l'embout 34, 35 qu'elle porte et sert en outre de butée de positionnement dans le tube (4, 16, 24, etc...) lors de l'insertion de l'embout dans un tube de fixateur, la coopération du méplat 52, 53 dudit segment de collerette 40, 41, coopérant avec le dégagement 38, 39 de la face interne de l'embout 34, 35 pour assurer une rotation, sans frottement des deux parties qui constituent la rotule 25, l'une par rapport à l'autre.

Une rotule 25 présentant l'agencement qui vient d'être décrit, permet d'orienter les tubes des fixateurs adjacents à assembler, dans tous les plans et permet en outre une rotation de la rotule 25 de 360° dans les tubes.

Selon un mode de réalisation de la rotule, particulièrement adapté aux cas où il est nécessaire d'articuler entre eux trois tubes par leurs extrémités respectives, par exemple dans le cas de l'arthrodèse du pied, c'est-à-dire de fractures tibio-tarsiennes, la rotule 42 (cf. figures 8 et 9) se compose des deux demi-sphères 29, 30 décrites plus haut avec leur embout 34, 35, entre lesquelles est insérée une pièce intermédiaire 43 qui se compose d'un élément 44 sensiblement circulaire, de même diamètre que les faces crantées 32, 33 de chaque demi-sphère, et dont les deux faces 45, 46 sont crantées pour permettre le blocage des faces 32-45-33-46 crantées en regard les unes des autres, par accrochage, lors de l'assemblage des pièces constitutives de la rotule 42. L'élément 44 porte un embout 47 dont la face tournée vers l'élément 44 est dégagée en 48-49, symétriquement par rapport au plan axial de l'élément 44 et de l'embout 47. L'embout 47 comporte, en outre, une gorge périphérique 50 destinée à recevoir l'extrémité d'une vis de blocage (non représentée) de l'embout dans l'extrémité d'un des tubes de fixateur à assembler.

L'élément 44 comporte en outre un segment de collerette 51 qui a pour rôle à la fois de relier l'embout 47 à l'élément 44 et de servir de butée de positionnement lors de l'insertion de l'embout 47 dans l'extrémité d'un tube de fixateur à assembler.

Les trois pièces qui constituent la rotule 42 sont assemblées entre elles par un axe 54 autour duquel chacune d'elles peut effectuer une rotation de 360°.

Une autre possibilité d'accouplement de deux tubes fixateurs adjacents, qui permet une rotation des tubes accouplés de 360° l'un par rapport à l'autre et permet, en outre, le décalage des tubes fixateurs, est représentée à la figure 11. Il s'agit d'un agencement comportant deux colliers 55 analogues aux colliers 17, 18, 19, 20 (cf. figures 4, 5 et 10), montés sur un axe commun 56, par l'intermédiaire d'orifices traversants (non visibles à la figure 11) ménagés dans les mors 57, 58 de chacun des deux colliers 55 associés. Les deux sorties symétriques intérieures des orifices traversants ménagés dans les mors 58 symétriques sont bordées d'un crantage 59 périphérique qui permet de bloquer les colliers 55 en position l'un par rapport à l'autre, sur leur axe 56 commun, en partie fileté 61, qui agit en compression sur les mors 57-58 du collier porté par la partie filetée 61 de l'axe 56, pour bloquer les deux colliers accouplés.

Un tel agencement est particulièrement intéressant dans les montages où il est nécessaire d'avoir une rigidité totale et où l'encombrement dû au décalage des tubes l'un par rapport à l'autre, est tolérable.

Un tel dispositif d'accouplement à deux colliers permet une adaptation aux besoins successifs dans le temps d'un même malade, sans avoir à changer d'appareillage, car l'un des tubes fixateurs peut, à volonté, être placé en position décalée haute ou basse, par exemple, en fonction des problèmes à résoudre, tels qu'une enflure, qui détermine de façon provisoire la mise en position haute d'un tube fixateur considéré, tandis que lorsque l'enflure disparaît, le tube fixateur est mis en position basse, en tenant compte, lors de ces déplacements successifs, de la donnée chirurgicale selon laquelle un fixateur doit être aussi proche que possible de l'os dont on cherche à réduire la fracture.

Les figures 12 et 13 représentent un collier 17, 18, 19 ou 20 dont la tête de l'écrou de blocage 60 présente un orifice traversant 62 perpendiculaire à l'axe de l'écrou 60 et dans lequel est insérée une douille 63 de réduction qui reçoit, par exemple, une broche 6. La douille 63 présente avantageusement des propriétés élastiques qui lui sont conférées par la disposition d'une fente 64 longitudinale. La douille 63 peut être orientée angulairement par rotation pour réaliser, par l'intermédiaire de la broche 6 qui y est insérée, l'immobilisation d'un fragment d'os qui ne passe pas dans le plan du tube et se trouve, par conséquent, hors du système de réduction du fixateur qui porte le collier auquel est associée la douille de réduction 63. Un tel agencement présente un intérêt considérable dans le cas de la réduction de fractures multiples se trouvant dans des plans extérieurs au(x) fixateur(s).

Ainsi que cela ressort de ce qui précède, l'invention ne se limite nullement à ceux de ses modes de réalisation et d'application qui viennent d'être décrits de façon plus explicite; elle en embrasse au contraire toutes les variantes qui peuvent venir à l'esprit du technicien en la matière, sans s'écarter du cadre, ni de la portée, de la présente invention telle que définie dans les revendications jointes.

**Revendications**

1. Appareil orthopédique pour immobiliser un os fracturé (1) du type comportant au moins un fixateur externe rigide (3), constitué par un tube perforé qui est placé à l'extérieur et au contact d'un membre ayant un os fracturé, et des broches autoforantes (6) qui sont vissées dans l'os fracturé, de part et d'autre de la fracture et qui sont engagées dans les perforations dudit tube et bloquées dans celui-ci par des vis de blocage (10), caractérisé en ce que ledit fixateur externe comprend deux tubes perforés distincts (4, 11) disposés selon le même axe dans le prolongement l'un de l'autre et reliés entre eux par un tube de liaison (12), en ce que l'un des deux tubes perforés (4) est solidarisé avec le tube de liaison (12) pour être fixe, tandis que l'autre tube perforé (11) peut être déplacé axialement le long du tube de liaison (12), par rapport au tube perforé fixe (4), en ce que le tube de liaison (12) porte un filetage extérieur au moins à ses deux extrémités opposées (12a, 12b) et en ce que deux écrous (14, 15) sont prévus respectivement sur les extrémités filetées (12a, 12b) du tube de liaison (12), au voisinage des extrémités correspondantes du tube perforé mobile (11), de manière à permettre le déplacement du tube mobile (11) le long du tube (12) et à bloquer ledit tube mobile (11) dans la position voulue, par vissage desdits écrous (14, 15) sur lesdites extrémités filetées du tube (12).

2. Appareil orthopédique selon la revendication 1, caractérisé en ce que le tube fixe (4) est solidarisé avec le tube de liaison (12) par vissage de l'une de ses extrémités filetées (12a notamment) dans le filetage interne prévu à l'extrémité correspondante du tube perforé fixe (4).

3. Appareil orthopédique selon la revendication 1, caractérisé en ce que le tube perforé fixe (4) est solidarisé avec le tube de liaison (12) par soudage de leurs extrémités adjacentes.

4. Appareil orthopédique selon la revendication 1, caractérisé en ce que le tube perforé fixe (4) et le tube de liaison (12) sont réalisés en une seule pièce.

5. Appareil orthopédique selon l'une quelconque des revendications 1 à 4, caractérisé en ce que ledit tube de liaison (12) comporte deux fentes longitudinales (16a, 16b) diamétralement opposées à travers lesquelles passent lesdites broches (6) et une ou deux fentes longitudinales (16c, 16d) situées dans un plan perpendiculaire au plan des deux premières fentes, dans l'une desquelles les vis de blocage (10) des broches sont engagées.

6. Appareil orthopédique selon l'une quelconque des revendications 1 à 5, caractérisé en ce que ledit fixateur externe (3) coopère avec un deuxième fixateur externe qui est disposé lui aussi à l'extérieur dudit premier membre ayant un os fracturé (1) et parallèlement au premier fixateur.

7. Appareil selon la revendication 6, caractérisé en ce que le deuxième fixateur externe est identique audit premier fixateur.

8. Appareil orthopédique selon la revendication 6, caractérisé en ce que le deuxième fixateur externe est constitué par un tube unique (16) perforé, qui coopère avec lesdites broches autoforantes (6) et qui est orienté de telle sorte que les broches (6) engagées dans les deux fixateurs sont situées dans deux plans faisant entre eux un angle (α) compris entre 60° et 120°, et en ce que les deux fixateurs sont reliés entre eux par des tiges transversales (22).

9. Appareil orthopédique selon l'une quelconque des revendications 6 à 8, caractérisé en ce que chacun desdits premier et deuxième fixateurs porte deux colliers coulissants (17, 18 et 19, 20), et en ce que lesdites tiges transversales (22) sont engagées et bloquées dans deux colliers situés respectivement sur chacun des deux fixateurs sensiblement au même niveau.

10. Appareil selon l'une quelconque des revendications 1 à 9, caractérisé en ce qu'il comporte un troisième fixateur rigide (24), qui est situé à l'extérieur d'une autre partie dudit membre comportant un deuxième os fracturé (23) et qui est relié à l'un desdits premier ou deuxième fixateurs situés à l'extérieur d'une première partie dudit membre comportant un premier os fracturé (1), par l'intermédiaire d'une rotule (25) située au niveau de l'articulation entre les deux os fracturés (1, 23).

11. Appareil orthopédique selon la revendication 9, caractérisé en ce que chaque collier (17, 18, 19, 20) se compose de deux parties sensiblement symétriques articulées entre elles et d'un dispositif de blocage de chaque collier (17, 18, 19, 20) en position voulue sur le tube perforé correspondant (4, 11, 16).

12. Appareil orthopédique selon la revendication 10, caractérisé en ce que la rotule (25) comprend deux parties orientables l'une par rapport à l'autre.

13. Appareil orthopédique selon la revendication 12, caractérisé en ce que les surfaces en regard, de chacune des deux parties dont se compose ladite rotule (25), qui sont sensiblement constituées par deux demi-sphères (29, 30), sont des surfaces crantées (32, 33) pour assurer le blocage mutuel desdites deux parties.

14. Appareil orthopédique selon l'une quelconque des revendications 12 et 13, caractérisé en ce que lesdites deux parties sont mobiles par rotation l'une par rapport à l'autre autour d'un axe (31) d'assemblage perpendiculaire à leurs surfaces de jonction (32, 33).

15. Appareil orthopédique selon l'une quelconque des revendications 12 à 14, caractérisé en ce que chacune desdites deux parties dont se compose la rotule comporte un embout (34, 35) de fixation dont chacun est destiné à être inséré dans l'une des extrémités de tubes (4, 11, 16, 24, etc...) voisins à relier suivant une orientation angulaire relative voulue.

16. Appareil orthopédique selon la revendication 15, caractérisé en ce que chaque embout comporte une gorge périphérique (36, 37) qui coopère avec une vis de blocage insérée dans l'un des trous ménagés sur la paroi d'un tube (4, 11, 16, 24, etc...) à relier, au voisinage de son extrémité, pour bloquer ledit embout en position dans l'extrémité dudit tube.

17. Appareil orthopédique selon l'une quelconque des revendications 15 et 16, caractérisé en ce que l'embout présente sur sa face tournée vers la partie de la rotule à laquelle il est associé, un dégagement (38, 39) propre à permettre la rotation de ladite partie de la rotule sans frottement.

18. Appareil orthopédique selon l'une quelconque des revendications 15 à 17, caractérisé en ce que l'embout est relié à la partie de la rotule à laquelle il est associé, par un segment de collerette (40, 41) comportant un méplat (52, 53) sensiblement dans le prolongement de la surface de jonction de ladite partie avec une surface en regard de l'autre partie de la rotule.

19. Appareil orthopédique selon la revendication 10, caractérisé en ce que la rotule se compose de trois parties, à savoir les deux parties (29, 30) selon l'une quelconque des revendications 12 à 18, et une pièce intermédiaire (44) annulaire de même diamètre que lesdites deux demi-sphères, dont les deux faces opposées (45, 46) sont également crantées et qui porte également un embout (47) conforme aux revendications 15 à 18.

20. Appareil orthopédique selon la revendication 9 et la revendication 11, caractérisé en ce que chacun des colliers (17, 18, 19, 20) se compose de deux demi-mâchoires (26, 27) articulées entre elles par une articulation à charnière (28) et en ce que le mors de l'une des demi-mâchoires porte une vis (21), notamment du type imperdable, et le mors de l'autre demi-mâchoire porte un écrou (60) qui coopère avec ladite vis pour bloquer ledit collier en position sur un tube (4, 11, 16, 24, etc...).

21. Appareil orthopédique selon la revendication 20, caractérisé en ce que le mors de la demi-mâchoire qui porte l'écrou de blocage (60) comporte sur sa face externe un dégagement pour loger la tête de l'écrou de blocage.

22. Appareil orthopédique selon la revendication 20, caractérisé en ce que la tête de l'écrou de blocage (60) comporte un orifice traversant (62) ménagé perpendiculairement à l'axe dudit écrou et destiné à recevoir une douille (63) propre à loger, notamment, une broche (6) destinée à immobiliser, dans le cas, en particulier, d'une fracture multiple, un fragment d'os se trouvant hors du système de réduction du fixateur.

23. Appareil orthopédique selon la revendication 9, la revendication 11 et l'une quelconque des revendications 20 et 21, caractérisé en ce qu'il comprend deux colliers (55) montés par leurs mors sur un axe commun (56), en ce que les faces adjacentes des mors (58) voisins de chaque collier (55) comportent chacune sur le pourtour des orifices de passage dudit axe, un crantage (59) d'accouplement desdits colliers en position bloquée, et en ce que ledit axe (56) est pourvu d'un écrou ou analogue de serrage en position des mors des colliers sur leur axe commun, pour bloquer les colliers dans n'importe quelle position relative l'un par rapport à l'autre.

24. Appareil orthopédique selon la revendication 22, caractérisé en ce que la douille de réduction (63) est pourvue de moyens lui conférant des propriétés élastiques.

**Claims**

1. Orthopedic apparatus for immobilizing a fractured bone (1) of the type comprising at least one rigid external fixing means (3) formed by a perforated tube which is placed on the outside of and in contact with the limb having a fractured bone and self-tapping pins (6) which are screwed into the fractured bone on each side of the fracture and which are engaged in the perforations of said tube and locked therein by locking screws (1), characterized in that said external fixing means comprises two separated perforated tubes (4, 11) disposed along the same axis in the extension of each other and connected together by a connecting tube (12), and in that one of said perforated tubes (4) is secured to said connecting tube (12) so as to be fixed, whereas the other perforated tube (11) may be moved axially along the connecting tube (12), with respect to the fixed perforated tube (4), in that the connecting tube (12) has an external threaded portion at least at its two opposite ends (12a, 12b), and in that two nuts (14, 15) are provided respectively on the threaded ends (12a, 12b) of the connecting tube (12), in the vicinity of the corresponding ends of the mobile perforated tube (11), so as to allow the mobile tube (11) to move along the tube (12) and to lock said mobile tube (11) in the desired position, by screwing said nuts (14, 15) on said threaded ends of the tube (12).

2. Orthopedic apparatus according to claim 1, characterized in that the fixed tube (4) is secured to the connecting tube (12) by screwing one of the threaded ends (12a particularly) into the internal threaded portion provided at the corresponding end of the fixed perforated tube (4).

3. Orthopedic apparatus according to claim 1, characterized in that the fixed perforated tube (4) is secured to the connecting tube (12) by welding their adjacent ends.

4. Orthopedic apparatus according to claim 1, characterized in that the fixed perforated tube (4) and the connecting tube (12) are made as a single piece.

5. Orthopedic apparatus according to any one of claims 1 to 4, characterized in that said connecting tube (12) has two diametrically opposite longitudinal slits (16, 16b) through which said pins (6) pass and one or two longitudinal slits (16c, 16d) situated in a plane perpendicular to the plane of the first slits, in which the locking screws (10) of the pins are engaged.

6. Orthopedic apparatus according to any one of claims 1 to 5, characterized in that said external fixing means (3) cooperate with second external fixing means which are also disposed outside said first limb having a fractured bone (1) and parallel to the first fixing means.

7. Orthopedic apparatus according to claim 6, characterized in that the second external fixing means is identical to said first fixing means.

8. Orthopedic apparatus according to claim 6, characterized in that the second external fixing means is formed by a single perforated tube (16) which cooperates with said self-tapping pins (6) and which is oriented so that the pins (6) engaged in the two fixing means are situated in two planes forming therebetween an angle (α) between 60° and 120°, and in that the two fixing means are connnected together by transverse rods (22).

9. Orthopedic apparatus according to any one of claims 6 to 8, characterized in that each of said first and second fixing means has two sliding collars (17, 18 and 19, 20) and in that said transverse rods (22) are engaged and locked in two collars situated respectively on each of the two fixing means substantially at the same level.

10. Orthopedic apparatus according to any one of claims 1 to 9, characterized in that it comprises a third rigid fixing means (24) which is situated outside another part of said limb having a second fractured bone (23) and which is connected to one of said first or second fixing means situated outside a first part of said limb having a first fractured bone (1), by means of a ball-joint (25) situated at the level of the joint between the two fractured bones (1, 23a).

11. Orthopedic apparatus according to claim 9, characterized in that each collar (17, 18, 19, 20) is formed of two substantially symmetrical parts hinged together and a device for locking each collar (17, 18, 19, 20) in the desired position on the corresponding perforated tube (4, 11, 16).

12. Orthopedic apparatus according to claim 10, characterized in that the ball-joint (25) comprises two parts which can swivel with respect to each other.

13. Orthopedic apparatus according to claim 12, characterized in that the facing surfaces of each of the two parts from which said ball-joint (25) is formed, which are substantially formed by two semispherres (29, 30), are toothed surfaces (32, 33) for the mutual blocking of said two parts.

14. Orthopedic apparatus according to any one of claims 12 and 13, characterized in that said two parts are rotatable with respect to each other about an assembly pin (31) perpendicular to their junction surfaces (32, 33).

15. Orthopedic apparatus according to any one of claims 12 to 14, characterized in that each of said two parts from which the ball-joint (25) is formed comprises a securing end piece (34, 35) each of which is intended to be inserted in one of the ends of adjacent tubes (4, 11, 16, 24, etc...) to be connected together in a desired relative angular orientation.

16. Orthopedic apparatus according to claim 15, characterized in that each end piece comprises a peripheral groove (36, 37) which cooperates with a locking screw inserted in one of the holes formed in the wall of a tube (4, 11, 16, 24, etc...) to be connected, in the vicinity of its end, for locking said end piece in position in the end of said tube.

17. Orthopedic apparatus according to any one of claims 15 and 16, characterized in that the end piece has, on its face turned towards the part of the ball-joint with which it is associated, a recessed part (38, 39) for permitting rotation of said part of the balljoint without friction.

18. Orthopedic apparatus according to any one of claims 15 to 17, characterized in that the end piece is connected to the part of the ball-joint with which it is associated by a collar segment (40, 41) comprising a land (52, 53) substantially in the extension of the junction surface of said part with a facing surface of the other part of the ball-joint.

19. Orthopedic apparatus according to claim 10, characterized in that the ball-joint is formed of three parts, namely the two parts (29, 30) according to any one of claims 12 to 18, and an intermediate annular part (44) of the same diameter as said two semi-spheres, whose two opposite faces (45, 46) are also toothed and which also comprises an end piece (47) in accordance with claims 15 to 18.

20. Orthopedic apparatus according to claim 9 and claim 11, characterized in that each of the collars (16, 18, 19, 20) is formed of two half grips (26, 27) hinged together by a hinge (28) and in that the jaw of one of the half grips carries a screw (21) particularly of the type that cannot be lost, and the jaw of the other half grip carries a nut (60) which cooperates with said screw for locking said collar in position on a tube (4, 11, 16, 24, etc...).

21. Orthopedic apparatus according to claim 20, characterized in that the jaw of the half grip which carries the locking nut (60) has a recess on its external face for housing the head of the locking nut.

22. Orthopedic apparatus according to claim 20, characterized in that the head of the locking nut (60) comprises a through-hole (62a formed perpendicularly to the axis of said nut for receiving a sleeve (63) adapted for housing, more particularly, a pin (6) for immobilizing, particularly in the case of a multiple fracture, a bone fragment situated outside the reduction system of the fixing means.

23. Orthopedic apparatus according to claim 9, claim 11 and any one of claims 20 and 21, characterized in that it comprises two collars (55) mounted by their jaws on a common shaft (56), in that the adjacent faces of the adjacent jaws (58) of each collar (55) each has, on the periphery of the apertures for passing the shaft, teeth (59) for coupling said collars in the locked position, and in that said shaft (56) is provided with a nut or similar for clamping the jaws of the collars in position on their comon shaft, for locking the collars in any position relative to each other.

24. Orthopedic apparatus according to claim 22, characterized in that the reduction sleeve (63) is provided with means conferring resilient properties thereon.

**Patentansprüche**

1. Orthopädische Vorrichtung zum Ruhigstellen eines gebrochenen Knochens (1) mit mindestens einer starren äußeren Halterung (3), die von einem perforierten (durchbohrten) Rohr, das außen und in Kontakt mit einem Glied mit gebrochenem Knochen angeordnet ist sowie selbstbohrenden Stiften (6) gebildet wird, die auf beiden Seiten des Bruches in den gebrochenen Knochen geschraubt sind und die in die Perforationen des Rohres eingreifen und darin durch Sicherungsschrauben (10) gesichert sind, dadurch gekennzeichnet, daß die äußere Halterung zwei verschiedene perforierte Rohre (4, 11) umfaßt, die in der Verlängerung voneinander in der gleichen Achse angeordnet und mittels eines Verbindungsrohres (12) so miteinander verbunden sind, daß eines (4) der perforierten Rohre mit dem Verbindungsrohr zur Befestigung verbunden ist, während das andere (11) perforierte Rohr axial längs des Verbindungsrohres (12) mit Bezug auf das fixierte perforierte Rohr (4) verschiebbar ist, wobei das Verbindungsrohr (12) an mindestens seinen beiden gegenüberliegenden Enden (12a, 12b) ein Außengewinde trägt und zwei Muttern (14, 15) auf den jeweiligen Gewindeenden (12a, 12b) des Verbindungsrohres (12) in der Nähe der entsprechenden Enden des beweglichen perforierten Rohres (11) derart vorgesehen sind, daß sie die Verschiebung des beweglichen Rohres (11) längs des Rohres (12) und die Sicherung des beweglichen Rohres (11) in der gewünschten Position durch Aufschrauben der Muttern (14, 15) auf die Gewindeenden des Rohres (12) gestatten.

2. Orthopädische Vorrichtung nach Anspruch 1, dadurch gekennzeichnet, daß das feste Rohr (4) mit dem Verbindungsrohr (12) dadurch verbunden ist, daß eines seiner Gewindeenden (insbesondere 12a) in ein Innengewinde geschraubt ist, das an dem entsprechenden Ende des festen perforierten Rohres (4) vorgesehen ist.

3. Orthopädische Vorrichtung nach Anspruch 1, dadurch gekennzeichnet, daß das feste perforierte Rohr (4) mit dem Verbindungsrohr (12) durch Verschweißen ihrer benachbarten Enden verbunden ist.

4. Vorrichtung nach Anspruch 1, dadurch gekennzeichnet, daß das feste perforierte Rohr (4) und das Verbindungsrohr (12) einstückig ausgebildet sind.

5. Orthopädische Vorrichtung nach irgendeinem der Ansprüche 1 bis 4, dadurch gekennzeichnet, daß das Verbindungsrohr (12) zwei diametral gegenüberliegende Längsschlitze (16a, 16b), durch die sich die genannten Stifte (6) erstrecken sowie ein oder zwei Längsschlitze (16c, 16d) aufweist, die sich in einer Ebene senkrecht zur Ebene der beiden erstgenannten Schlitze befinden und in deren einen die Sicherungsschrauben (10) der Stifte eingreifen.

6. Orthopädische Vorrichtung nach irgendeinem der Ansprüche 1 bis 5, dadurch gekennzeichnet, daß die äußere Halterung (3) mit einer zweiten äußeren Halterung zusammenarbeitet, die ebenfalls außen an dem Glied mit einem gebrochenen Knochen (1) und parallel zur ersten Halterung angeordnet ist.

7. Vorrichtung nach Anspruch 6, dadurch gekennzeichnet, daß die zweite äußere Halterung identisch der genannten ersten Halterung ist.

8. Orthopädische Vorrichtung nach Anspruch 6, dadurch gekennzeichnet, daß die zweite äußere Halterung durch ein einziges perforiertes Rohr (16) gebildet wird, das mit den genannten selbstbohrenden Stiften (6) zusammenarbeitet und das derart orientiert ist, daß die Stifte (6), die in die beiden Halterungen eingreifen, in zwei Ebenen angeordnet sind, die einen Winkel ($\alpha$) zwischen 60° und 120° bilden, wobei die beiden Halterungen durch Querstangen (22) miteinander verbunden sind.

9. Orthopädische Vorrichtung nach irgendeinem der Ansprüche 6 bis 8, dadurch gekennzeichnet, daß jede der beiden Halterungen zwei verschiebbare Ringe (17, 18 und 19, 20) trägt, in denen die genannten Querstangen (22) eingreifen und gesichert sind wobei die beiden Ringe auf jeder der beiden Halterungen jeweils im wesentlichen in der gleichen Höhe angeordnet sind.

10. Vorrichtung nach irgendeinem der Ansprüche 1 bis 9, dadurch gekennzeichnet, daß sie eine dritte starre Halterung (24) aufweist, die außen an einem anderen Teil des Gliedes angeordnet ist, das einen zweiten gebrochenen Knochen (23) hat und die mittels eines Kugelgelenks (25), das in der Höhe des Gelenkes zwischen den beiden gebrochenen Knochen (1, 23) angeordnet ist, mit einer der beiden erstgenannten Halterungen verbunden ist, die sich außen an einem ersten Teil des genannten Gliedes mit einem ersten gebrochenen Knochen (1) befinden.

11. Orthopädische Vorrichtung nach Anspruch 9, dadurch gekennzeichnet, daß jeder Ring (17, 18, 19, 20) aus zwei im wesentlichen symmetrischen Teilen, die beweglich miteinander verbunden sind und einer Einrichtung zum Feststellen jedes Ringes (17, 18, 19, 20) in gewünschter Position auf dem entsprechenden perforierten Rohr (4, 11, 16) zusammengesetzt sind.

12. Orthopädische Vorrichtung nach Anspruch 10, dadurch gekennzeichnet, daß das Kugelgelenk (25) zwei zueinander orientierbare Teile umfaßt.

13. Orthopädische Vorrichtung nach Anspruch 12, dadurch gekennzeichnet, daß die sich gegenüberstehenden Oberflächen jedes der beiden Teile, aus denen sich das Kugelgelenk (25) zusammensetzt, die im wesentlichen durch zwei Halbkugeln (29, 30) gebildet werden, gerippte Oberflächen (32, 33) sind, um das gegenseitige Feststellen der beiden Teile sicherzustellen.

14. Orthopädische Vorrichtung nach irgendeinem der Ansprüche 12 und 13, dadurch gekennzeichnet, daß die beiden Teile gegeneinander um eine Montageachse (31) drehbar sind, die senkrecht zu den verbundenen Oberflächen (32, 33) verläuft.

15. Orthopädische Vorrichtung nach irgendeinem der Ansprüche 12 bis 14, dadurch gekennzeichnet, daß jedes der beiden Teile, aus denen sich das Kugelgelenk zusammensetzt, einen Befestigungsansatz (34, 35) aufweist, deren jeder dazu bestimmt ist, in eines der Enden benachbarter, in einer gewünschten relativen Winkelorientierung zu verbindender Rohre (4, 11, 16, 24 etc.) eingeführt zu werden.

16. Orthopädische Vorrichtung nach Anspruch 15, dadurch gekennzeichnet, daß jeder Ansatz eine periphere Nut (36, 37) aufweist, die mit einer Sicherungsschraube zusammenarbeitet, die in ein nahe seinem Ende in der Wand eines zu verbindenden Rohres (4, 11, 16, 24 etc.) ausgespartes Loch eingeführt ist, um den Ansatz in seiner Position im Ende des genannten Rohres zu sichern.

17. Ortopädische Vorrichtung nach irgendeinem der Ansprüche 15 und 16, dadurch gekennzeichnet, daß der Ansatz auf seiner dem Teil des Kugelgelenkes zugewandten Fläche, mit dem er verbunden ist, eine geeignete Trenneinrichtung (38, 39) aufweist, um die Rotation des genannten Teiles des Kugelgelenkes ohne Reibung zu gestatten.

18. Orthopädische Vorrichtung nach irgendeinem der Ansprüche 15 bis 17, dadurch gekennzeichnet, daß der Ansatz mit dem Teil des Kugelgelenkes, mit dem er vereinigt ist, durch ein Kragensegment (40, 41) verbunden ist, das eine Abflachung (52, 53) umfaßt, die sich im wesentlichen in der Verlängerung der Verbindungsoberfläche des genannten Teiles mit einer gegenüberstehenden Oberfläche des anderen Teiles des Kugelgelenkes befindet.

19. Orthopädische Vorrichtung nach Anspruch 10, dadurch gekennzeichnet, daß das Kugelgelenk sich aus drei Teilen zusammensetzt, nämlich den beiden Teilen (29, 30) nach irgendeinem der Ansprüche 12 bis 18 und einem ringförmigen Zwischenstück (44) mit gleichem Durchmesser wie dem der beiden Halbkugeln und dessen beide gegenüberliegende Flächen (45, 46) gleichermaßen gerippt sind und das gleichermaßen einen Ansatz (47) gemäß den Ansprüchen 15 bis 18 trägt.

20. Orthopädische Vorrichtung nach Anspruch 9 und Anspruch 11, dadurch gekennzeichnet, daß jeder der Ringe (17, 18, 19, 20) sich aus zwei Halbklemmbacken (26, 27) zusammensetzt, die durch ein Scharniergelenk (28) schwenkbar miteinander verbunden sind, wobei die Spannbacke der einen halben Klemmbacke eine Schraube (21) trägt, insbesondere eine solche, die unverlierbar ist und die Spannbacke der anderen halben Klemmbacke eine Mutter (60) trägt, die mit der genannten Schraube zusammenarbeitet, um den Ring in seiner Position auf dem Rohr (4, 11, 16, 24 etc.) festzulegen.

21. Orthopädische Vorrichtung nach Anspruch 20, dadurch gekennzeichnet, daß die Spannbacke der Halbklemmbacke, die die Feststellmutter (60) trägt, auf ihrer Außenfläche eine Freigabeeinrichtung zum Einsetzen des Kopfes der Feststellmutter aufweist.

22. Orthopädische Vorrichtung nach Anspruch 20, dadurch gekennzeichnet, daß der Kopf der Feststellmutter (60) eine durchgehende Öffnung (62) aufweist, die senkrecht zur Achse der Mutter verläuft und zur Aufnahme einer geeigneten Hülse (63) bestimmt ist, die insbesondere einen Stift (6) aufnimmt, der insbesondere im Falle eines Mehrfachbruches ein Knochenfragment ruhigstellen soll, das sich außerhalb des Rückführungssystems der Halterung befindet.

23. Orthopädische Vorrichtung nach Anspruch 9, Anspruch 11 und irgendeinem der Ansprüche 20 und 21, dadurch gekennzeichnet, daß es zwei Ringe (55) umfaßt, die über ihre Spannbacken auf einer gemeinsamen Achse (56) montiert sind, wobei die aneinanderliegenden Flächen der benachbarten Spannbacken (58) jedes Ringes (55) jeweils auf dem Gang der durchgehenden Öffnungen der genannten Achse eine Einrastkupplung (59) für die genannten Ringe in festgestellter Position umfassen und wobei die genannte Achse (56) mit einer Mutter oder ähnlichem versehen ist, um die Spannbacken der Ringe auf ihrer gemeinsamen Achse in einer Position festzuspannen und die Ringe in irgendeiner beliebigen relativen position zueinander zu sichern.

24. Orthopädische Vorrichtung nach Anspruch 22, dadurch gekennzeichnet, daß die Reduzierhülse (63) mit Mitteln versehen ist, die ihr elastische Eigenschaften verleihen.

Fig. 1

Fig. 2

Fig. 3

0 140 786

Fig. 4

Fig. 5

5

FIG. 6

FIG. 8

FIG. 7

FIG. 9

FIG. 10

FIG. 11

FIG. 13

FIG. 12